# EUROPÄISCHE PATENTSCHRIFT

(11) **EP 1 145 725 B1**
(45) Veröffentlichungstag und Bekanntmachung des Hinweises auf die Patenterteilung: **23.08.2006**
(21) Anmeldenummer: 01107849.0
(22) Anmeldetag: 09.04.2001
(51) Int. Cl.: A61M 1/36, A61M 39/22

(54) **Herz-Lungen-Maschine mit druckmittelbetätigten Steuerorganen**
Cardio-pulmonary system comprising a fluid operated control member
Sytème cardiopulmonaire avec un organe de commande actionné par un fluide

(30) Priorität: 11.04.2000 DE 10017847
(43) Veröffentlichungstag der Anmeldung: 17.10.2001
(73) Patentinhaber: Sorin Group Deutschland GmbH, 80939 München (DE)
(72) Erfinder: Knott, Erwin, Dr., 85586 Poing (DE)
(74) Vertreter: HOFFMANN EITLE

(56) Entgegenhaltungen:
- EP-A- 0 659 444
- US-A- 4 250 872
- US-A- 4 925 152
- US-A- 5 814 004

## Beschreibung

Die Erfindung betrifft Herz-Lungen-Maschinen mit druckmittelbetätigten Steuerorganen.

Bei Herz-Lungen-Maschinen wird das Blut eines Patienten in einem extrakorporalen Kreislauf durch Schlauchleitungen und medizinische Geräte, beispielsweise einen Sauerstoffkonzentrator (Oxygenator) transportiert. Die Förderung des Blutes im extrakorporalen Kreislauf erfolgt mit Hilfe von Pumpen, insbesondere Rollerpumpen, bei denen der Rotor von außen auf einen Schlauchabschnitt einwirkt, der in den Stator der Rollerpumpe eingelegt ist. Indem die an dem Rotor angebrachten Rollen auf dem Schlauchabschnitt abrollen, wird durch Verdrängung das in der Schlauchleitung geführte Blut mit einer Geschwindigkeit von bis zu 3 m/s gefördert.

Der extrakorporale Kreislauf muss gesteuert werden, was üblicherweise durch unmittelbare Beeinflussung der Pumpe und mit Hilfe von druckmittelbetätigten oder elektromechanischen Steuerungsorganen geschieht. Wenn druckmittelbetätigte Steuerungsorgane eingesetzt werden, müssen speziell angepasste Schlauchleitungen verwendet werden. So ist aus US 5,814,004 ein System zur Steuerung des Drucks in einem extrakorporalen Kreislauf bekannt, bei dem druckmittelbetätigte Ventile vorgesehen sind, um den Kreislauf zu beeinflussen. Auch bei diesem System sind die Schlauchleitungen an den Stellen, an denen die druckmittelbetätigten Steuerungsorgane vorgesehen sind, mit einer dünneren Schlauchwand versehen. Dadurch kann das Druckmittel des Steuerungsorgans mechanisch auf den Schlauch bis zu einem vollständigen Verschluss des Schlauchquerschnitts einwirken.

Nachteilig bei diesem und anderen bekannten Systemen für die Steuerung des extrakorporalen Kreislaufs einer Herz-Lungen-Maschine ist, dass speziell ausgebildete Schlauchleitungen verwendet werden müssen, um mit Hilfe der druckmittelbetätigten Steuerungsorgane den extrakorporalen Kreislauf zu beeinflussen. Die Schlauchleitungen müssen an den Stellen, an denen druckmittelbetätigte Steuerungsorgane einwirken sollen, mit einem veränderten Querschnitt und/oder einer verringerten Wanddicke ausgebildet werden oder aus einem anderen Material bestehen, was sowohl die Herstellung verteuert als auch die Handhabung erschwert.

Jedoch wird bislang dieser Nachteil in Kauf genommen oder auf elektromechanische Steuerungsorgane zurückgegriffen, die auch auf einfache Schlauchleitungen, d.h. nicht speziell für das Zusammenwirken mit den Steuerungsorganen vorbereitete Schlauchleitungen einwirken können. Die einfachen Schlauchleitungen können als "StandardSchlauchleitungen" bezeichnet werden; bei ihnen handelt es sich im wesentlichen um Schlauchleitungen mit konstantem Querschnitt und konstanter Wanddicke und mit gleichbleibender Materialzusammensetzung, also um Schlauchleitungen, wie sie beim Einsatz von Herz-Lungen-Maschinen üblicherweise verwendet werden. Diese einfachen Schlauchleitungen werden von den Anwendern bevorzugt, da sie in jeder erforderlichen Länge zur Verfügung sehen oder zurecht geschnitten werden können und weil die elektromechanischen Steuerungsorgane an jeder Stelle der Schlauchleitung angeordnet werden können.

Sowohl die elektromechanischen Steuerungsorgane als auch die druckmittelbetätigten Steuerungsorgane sind bislang für zeitkritische Vorgänge im extrakorporalen Kreislauf einer Herz-Lungen-Maschine nicht eingesetzt worden, insbesondere nicht um die Schlauchleitung in Notsituationen schlagartig zu verschließen, beispielsweise wenn ein Blasendetektor Luftblasen in dem im extrakorporalen Kreislauf geförderten Blut feststellt. Dies kann zum einen darauf zurückgeführt werden, dass elektromechanische Steuerungsorgane als nicht-zuverlässig genug angesehen werden und zum anderen darauf, dass die druckmittelbetätigten Steuerungsorgane nicht ausreichend schnell einen Verschluss der Schlauchleitung erreichen.

Aus DE 692 12 808 T2 ist ein Autotransfusionssystem bekannt, bei dem ein an Schlauchleitungen angeschlossenes Ventil vorgesehen ist, bei dem es sich um ein elektrisch, pneumatisch oder hydraulisch betriebenes Absperrventil handelt und das an einen elektrischen, optischen oder kapazitiven Pegelsensor angeschlossen ist. Letzterer ist innerhalb oder außerhalb eines Behälters angebracht, in dem Blut gesammelt wird, und steuert das Ventil bei einem bestimmten vorgegebenen hohen bzw. niedrigen Flüssigkeitspegel im Behälter öffnend oder schließend an. Da das Ventil in einer Schlauchleitung angeordnet ist, wird nicht auf die Schlauchleitung eingewirkt, um diese zu verschließen.

Eine manuell betätigbare Absperreinrichtung für Kunststoff-Schlauchleitungen ist aus DE 38 11 552 A1 bekannt. In einem nutförmigen Kanal wird ein rollenförmiges Verschiebeteil von Hand verschoben und dabei in eine den Kunststoffschlauch abklemmende Stellung bewegt. Diese Absperreinrichtung ist jedoch nicht druckmittelbetätigt, sondern wird von Hand bedient.

Aus EP 0 659 444 A1 (nächstliegender Stand der Technik) ist eine Vorrichtung zum Pumpen vom Blut bekannt, bei der eine Steuereinheit die Schließ- und Öffnungszeiten und -geschwindigkeiten von Klemmventilen steuert, die auf eine Schlauchleitung eines extrakorporalen Kreislaufs einwirken. Die Parameter der Steuereinheit sind variabel, um auf diesem Weg den künstlichen Blutkreislauf möglichst weitgehend dem natürlichen Kreislauf anzugleichen.

Vor diesem Hintergrund besteht das von der Erfindung zu lösende Problem darin, eine Herz-Lungen-Maschine mit druckmittelbetätigten Steuerungsorganen so auszubilden, dass Standardschlauchleitungen ohne für die Einwirkung der Steuerungsorgane speziell ausgebildete Abschnitte eingesetzt und mit Hilfe der Steuerungsorgane zuverlässig und schnell beeinflusst, insbesondere verschlossen werden können, so dass die Möglichkeit geschaffen wird, zeitkritische Vorgänge zu realisieren, wie beispielsweise das Verschließen einer Schlauchleitung nach der Erfassung einer Luftblase im extrakorporalen Kreislauf.

Gelöst wird diese Aufgabe durch eine Herz-Lungen-Maschine mit den Merkmalen gemäß Patentanspruch 1. Vorteilhafte Ausgestaltungen ergeben sich aus den Unteransprüchen.

In einer speziellen Ausgestaltung ist die erfindungsgemäße Herz-Lungen-Maschine so ausgebildet, dass auf die Erfassung einer Luftblase im extrakorporalen Kreislauf die Zuführung des geförderten Blutes zum Patienten unterbrochen wird, ohne dass die detektierte Luftblase zum Patienten gelangt.

Die Erfindung beruht auf der Erkenntnis, dass mit Hilfe eines druckmittelbetätigten Steuerungsorgans ohne weiteres eine ausreichende Energiedichte bereitgestellt werden kann, um eine Standardschlauchleitung zur Steuerung des extrakorporalen Kreislaufes zu beeinflussen, beispielsweise zu verschließen. Überraschenderweise findet sich im Stand der Technik kein Hinweis auf den Einsatz eines Druckmittels, dessen Betriebsdruck zwischen 2 und 10 bar liegt und damit die Möglichkeit eröffnet, üblicherweise verwendete Schlauchleitungen mit Hilfe eines druckmittelbetätigten Steuerungsorgans zu verschließen und dabei auch zeitkritische Vorgänge zu realisieren. Indem die Erfindung auf Druckmittel in dem angegebenen Betriebsdruckbereich zurückgreift, können übliche Schlauchleitungen, sogenannte Standardschlauchleitungen ohne weitere Veränderungen an Querschnitt, Wanddicke oder Material eingesetzt werden. Der im Vergleich mit dem Stand der Technik sehr hohe Betriebsdruck erlaubt eine mechanische Einwirkung auf den extrakorporalen Kreislauf, der nicht nur die grundsätzliche Möglichkeit der Steuerung des extrakorporalen Kreislaufs eröffnet, sondern dabei die Steuerung zeitkritischer Vorgänge mit einschließt. Dies ist darauf zurückzuführen, dass erst durch den Einsatz des Druckmittels im erfindungsgemäßen Betriebsdruckbereich von 2 bis 10 bar ein durch Formschluss definierter Schließzustand aufgrund der hohen Stellkräfte erreicht werden kann.

Besonders vorteilhaft ist daher, dass in Krankenhäusern regelmäßig ein Druckmittel mit einem Betriebsdruck von ca. 5 bar zur Verfügung steht, so dass die erfindungsgemäße Herz-Lungen-Maschine dort ohne zusätzlichen Aufwand bei der Druckmittelbereitstellung betrieben werden kann.

Vorteilhaft ist auch, dass das erfindungsgemäße Steuerungsorgan mit Druckmittelbetätigung nicht mit dem Blut des extrakorporalen Kreislaufs unmittelbar in Berührung kommt, da die Einwirkung stets von außen auf den nicht speziell präparierten Schlauch erfolgt.

Das erfindungsgemäß hohe Druckniveau des Druckmittels erlaubt lange Zuleitungen, was gerade für den klinischen Einsatz von Vorteil ist, wenn auf das zur Verfügung stehende Druckmittel mit einem Betriebsdruck von 5 bar zurückgegriffen wird. Die Herz-Lungen-Maschine gemäß der Erfindung kann aber auch mit einem Druckerzeuger oder einer Druckflasche ausgerüstet werden, die das Druckmittel den nötigen Betriebsdruck für das Druckmittel zur Betätigung der Steuerungsorgane bereitstellt.

In einer vorteilhaften Ausgestaltung sind die druckmittelbetätigten Steuerungsorgane Kolbenfedersysteme, weil insbesondere mit dieser Art von Steuerungsorganen sehr hohe Kräfte und sehr hohe Energien am Ort des Steuerungsorgans bereitgestellt werden können, wodurch eine besonders hohe Reaktionsschnelligkeit erreicht wird. Damit eignen sich als Kolbenfedersysteme ausgebildete Steuerungsorgane in ganz besonderem Maße für den Einsatz in zeitkritischen Systemen, beispielsweise im Zusammenwirken mit einem Blasendetektor.

Im folgenden wird die Erfindung anhand von Ausführungsbeispielen unter Bezugnahme auf die beigefügten Figuren näher erläutert. In den Figuren zeigt:
- Fig.1: den grundsätzlichen Aufbau einer Herz-Lungen-Maschine mit erfindungsgemäßen Steuerungsorganen; und
- Fig. 2A und 2B: ein Ausführungsbeispiel eines erfindungsgemäßen Steuerungsorgans.

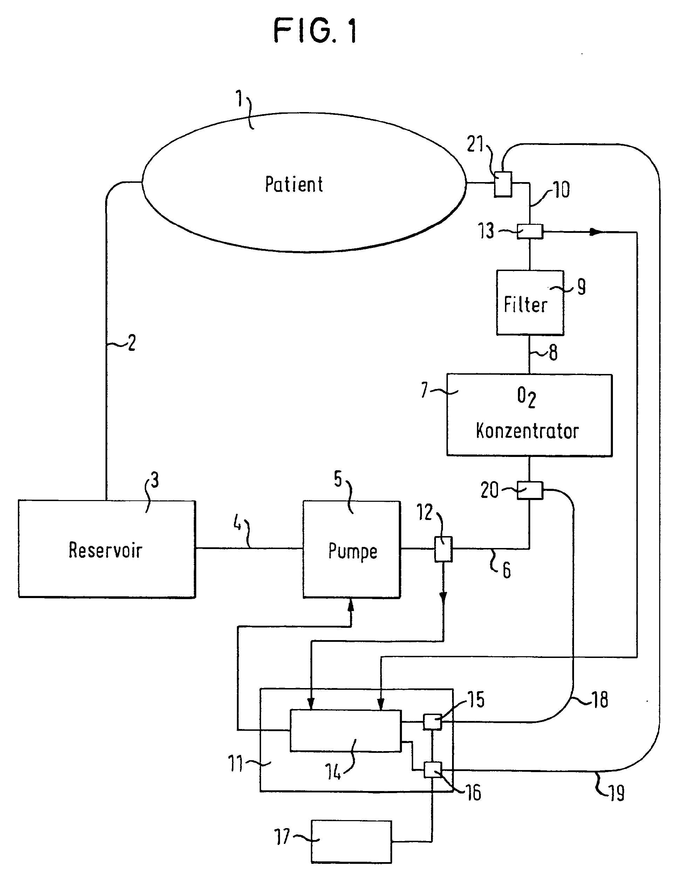

In Fig. 1 ist beispielhaft der extrakorporale Kreislauf einer erfindungsgemäßen Herz-Lungen-Maschine dargestellt. Der extrakorporale Kreislauf beginnt am Patienten 1 mit einer ersten Schlauchleitung 2, die über eine Kanüle venöses Blut des Patienten 1 abführt. Das Blut gelangt durch die Schlauchleitung 2 in ein Reservoir 3, an das eine zweite Schlauchleitung 4 angeschlossen ist. Durch die zweite Schlauchleitung 4 gelangt das Blut aus dem Reservoir 3 zur Pumpe 5, die das Blut durch die dritte Schlauchleitung 6 zu einem Sauerstoffkonzentrator (Oxigenator) 7 fördert. Von dort gelangt das Blut durch die vierte Schlauchleitung 8 zu einem Filter 9 bevor es durch die fünfte Schlauchleitung 10 und eine am Ende angeschlossene arterielle Kanüle dem Patienten 1 wieder zugeführt wird.

Die in Fig. 1 gezeigte erfindungsgemäße Herz-Lungen-Maschine besitzt darüber hinaus eine Steuerung 11, der Signale von einem ersten Blasendetektor 12 und einem zweiten Blasendetektor 13 zugeführt werden. Die Blasendetektoren 12 und 13 sind an der dritten Schlauchleitung 6 bzw. der fünften Schlauchleitung 10 angebracht und überwachen das in den Schlauchleitungen geförderte Blut im Hinblick auf das Auftreten von Luftblasen. Tritt in dem in den Schlauchleitungen geförderten Blut eine Luftblase auf, erzeugen die Blasendetektoren 12 und 13 ein Signal, das einem Prozessor 14 in der Steuerung 11 zugeführt wird. Der Prozessor 14 öffnet ein erstes Ventil 15, wenn er ein Signal des ersten Blasendetektors 12 empfängt, und das zweite Ventil 16, wenn er ein Signal des zweiten Blasendetektors 13 empfängt. Die beiden Ventile 15 und 16 sind mit einer Druckmittelabgabeeinrichtung 17 verbunden, bei der es sich um einen Druckspeicher, einen Kompressor oder dem Druckmittelanschluss eines Druckmittelversorgungsnetzes eines Krankenhauses o.ä. handeln kann. Das in der Druckmittelabgabeeinrichtung 17 vorhandene Druckmittel, wird über die Ventile 15 und 16 an eine erste Schlauchleitung 18 bzw. eine zweite Schlauchleitung 19 abgegeben, wenn das jeweilige Ventil geöffnet ist. Die Druckmittelschlauchleitungen 18 und 19 sind mit jeweils einem druckmittelbetätigtem Steuerungsorgan 20 und 21 verbunden. Das erste druckmittelbetätigte Steuerungsorgan 20 ist bezogen auf den ersten Blasendetektor 12 stromabwärts an der dritten Schlauchleitung 6 angeordnet. Das zweite druckmittelbetätigte Steuerungsorgan 21 ist bezogen auf den zweiten Blasendetektor 13 stromabwärts an der fünften Schlauchleitung 10 angeordnet.

Erfindungsgemäß stellt die Druckmittelbereitstellungseinrichtung 17 ein Druckmittel mit einem Betriebsdruck von 2 bis 10 bar, vorzugsweise 5 bar, bereit, mit dem die druckmittelbetätigten Steuerungsorgane betätigt werden, wenn das erste Ventil 15 bzw. das zweite Ventil 16 geöffnet wird. Aufgrund des sehr hohen Betriebsdrucks des Druckmittels, das in der Druckmittelbereitstellungseinrichtung 17 bereitgestellt wird, ist es erfindungsgemäß möglich, am Ort der druckmittelbetätigten Steuerungsorgane 20 und 21 hohe Energien mechanisch bereitzustellen, so dass schnelle Steuerungsvorgänge, insbesondere schnelle Schaltvorgänge realisierbar sind.

So ist es möglich, die Steuerung 11 so auszulegen, dass unmittelbar nach Erfassung einer Luftblase durch einen der Blasendetektoren 12 oder 13 der Prozessor 14 das entsprechende Ventil 15 oder 16 öffnet, um dem zu den Blasendetektor gehörenden Steuerungsorgan 20 bzw. 21 das erfindungsgemäß unter hohem Druck stehende Druckmittel über die Schlauchleitungen 18 oder 19 zuzuführen. Der erfindungsgemäß hohe Betriebsdruck führt zu einem schnellen Schaltverhalten mit entsprechend geringer Reaktionszeit, so dass die Erfassung einer Luftblase durch einen der Blasendetektoren einen sofortigen Verschluss der Schlauchleitung durch das stromabwärts am nächsten liegende druckmittelbetätigte Steuerungsorgan möglich ist. Vorzugsweise hält der Prozessor 14 dann auch die Pumpe an, wozu der Prozessor 14 mit der Pumpe 5 verbunden ist.

Der erfindungsgemäß hohe Betriebsdruck führt außerdem dazu, dass die Schaltung der Steuerungsorgane überaus zuverlässig bewirkt werden kann.

Die Zuverlässigkeit kann weiter erhöht werden, indem auf bewährte Steuerungsorgane aus anderen technischen Bereichen zurückgegriffen wird. Dies ist erfindungsgemäß möglich, da die Steuerungsorgane aufgrund des hohen Betriebsdrucks des Druckmittels unmittelbar auf Standardschlauchleitungen 2, 4, 6, 8 oder 10 einwirken können. Standardschlauchleitungen sind aber regelmäßig sehr robust, so dass beim Einsatz von erprobten Hochleistungssteuerungsorganen keine Beschädigung zu befürchten ist.

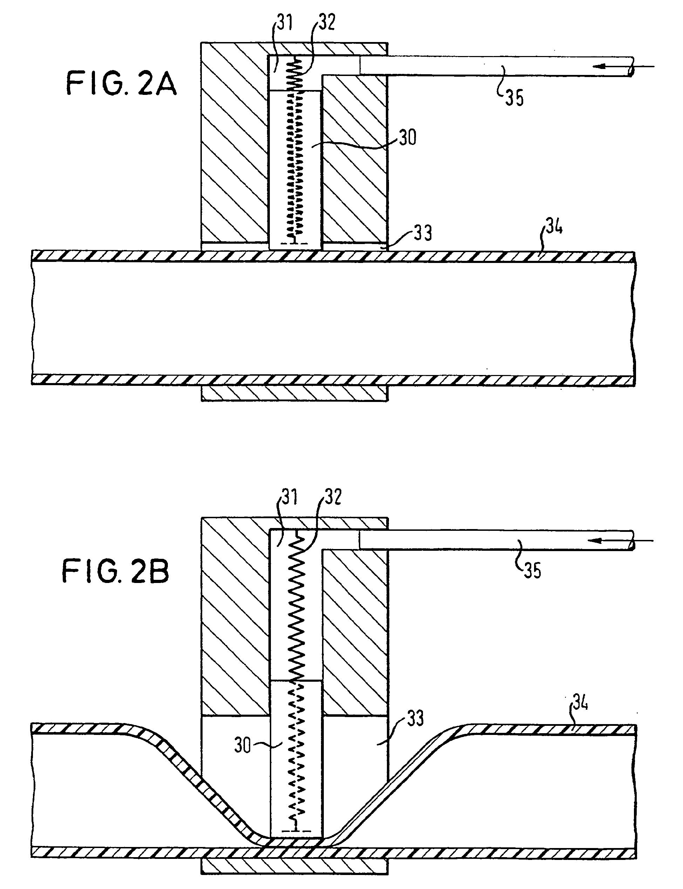

Besonders bewährt haben sich als druckmittelbetätigte Steuerungsorgane Kolbenfedersysteme. Wie in Fig. 2A und 2B gezeigt ist bei derartigen Systemen ein Kolben 30 in einem Zylinder 31 angeordnet und wird durch eine Feder 32 in einer Ruhelage (Fig. 2A) gehalten. In dieser Ruhelage wirkt der Kolben 30 nicht auf die in eine Aufnahme 33 des Steuerungsorgans eingelegte Schlauchleitung 34 ein. Wird über die Druckmittelleitung 35 dem Steuerorgan ein Druckmittel zugeführt, das erfindungsgemäß unter hohem Druck steht, wird der Kolben 30 sehr schnell und mit hoher Kraft gegen die Federvorspannung bewegt und die Standardschlauchleitung 34 verschlossen, wie in Fig. 2B dargestellt. Dabei ist zu beachten, dass als Schlauchleitung 34 eine Standardschlauchleitung oder nicht speziell für die Einwirkung des Steuerorgans vorbereitete Schlauchleitung verwendet wird. Deshalb kann, sofern der erfindungsgemäß hohe Betriebsdruck des Druckmittels bereitgestellt und eingesetzt wird, jede üblicherweise mit Herz-Lungen-Maschinen verwendete Schlauchleitung verwendet werden, ohne dass auf die druckmittelbetätigten Steuerungsorgane besondere Rücksicht genommen werden müsste.

Darüber hinaus wird durch den erfindungsgemäß hohen Betriebsmitteldruck gewährleistet, dass zeitkritische Vorgänge, beispielsweise der Verschluss der Schlauchleitung des extrakorporalen Kreislaufs bei Detektion einer Luftblase im Blut realisierbar ist. Besonders zu betonen ist dabei, dass der Abstand zwischen dem Blasendetektor und dem die Schlauchleitung verschließenden Steuerungsorgan vergleichsweise kurz gewählt werden kann, da bei den üblicherweise auftretenden Strömungsgeschwindigkeiten von 3 m/s des Blutes in der Schlauchleitung und der gleichzeitig hohen Reaktionsgeschwindigkeit der erfindungsgemäßen Steuerungsorgane keine Probleme auftreten.

Das erfindungsgemäß hohe Druckniveau des die Steuerungsorgane betätigenden Druckmittels bringt überdies den Vorteil, dass auch sehr lange Zuleitungen zu den druckmittelbetätigten Steuerungsorganen möglich sind.

Als Druckmittel kommen vorzugsweise Gase in Betracht. Der Einsatz von Gasen ist insbesondere bei Kolbenfedersystemen oder anderen ähnlichen Steuerungsorganen unproblematisch, da das Gas nicht unmittelbar auf die Standardschlauchleitung des extrakorporalen Kreislaufes einwirkt. Auch im Hinblick auf die Handhabbarkeit, insbesondere im Bereich eines OP sind gasförmige Druckmittel, wie z.B. Luft, Helium oder Stickstoff zu bevorzugen.

## Patentansprüche

1. Herz-Lungen-Maschine mit zumindest einer Schlauchleitung (6, 10; 34), die in einen extrakorporalen Blutkreislauf (2, 3, 4, 5, 6, 7, 8, 9, 10) der Herz-Lungen-Maschine integriert ist, mit zumindest einer Pumpe (5), die das Blut eines Patienten zumindest abschnittsweise durch den extrakorporalen Kreislauf fördert, zumindest einem druckmittelbetätigten Steuerungsorgan (20, 21; 30, 31,32, 33), das auf die Schlauchleitung (6, 10; 34) von außen einwirkt, und einer Steuerungseinrichtung (11) für Steuerung der Zuführung eines Druckmittels an das Steuerungsorgan,
**dadurch gekennzeichnet, dass**
am art des druckmittelbetätigtbaren Steuerorganes ein Druckmittel mit einem Betriebsdruck im Bereich von 2 bis 10 bar bereitgestellt wird.

2. Herz-Lungen Maschine nach Anspruch 1, **dadurch gekennzeichnet, dass** das dem druckmittelbetätigten Steuerorgan zugeführte Druckmittel einen Betriebsdruck im Bereich von ca. 5 bar aufweist.

3. Herz-Lungen-Maschine nach einem der Ansprüche 1 oder 2, **dadurch gekennzeichnet, dass** die Steuerung (11) mit einer Druckmittelabgabeeinrichtung (17) in Verbindung steht, und zumindest eine Ventileinrichtung (15, 16), der das Druckmittel von der Druckmittelabgabeeinrichtung zugeführt wird, und einen Prozessor (14) aufweist, der mit Ventileinrichtung (15, 16) zur deren Ansteuerung in Verbindung steht.

4. Herz-Lungen-Maschine nach einem der Ansprüche 1, 2 oder 3, **dadurch gekennzeichnet, dass** zumindest ein Blasendetektor (12, 13) zur Feststellung von Gasblasen in dem in der Schlauchleitung geführten Blutstrom bezogen auf den Blutstrom oberhalb von dem druckmittelbetätigten Steuerungsorgan (20, 21) vorgesehen ist, und dass der Prozessor (14) der Steuerung (11) mit dem Blasendetektor (12, 13) für den Empfang eines Detektorsignals in Verbindung steht, um das Ventil (20, 21) umzuschalten.

5. Herz-Lungen-Maschine nach Anspruch 4, **dadurch gekennzeichnet, dass** der Prozessor (14) beim Empfang eines Detektorsignals von dem Blasendetektor (20, 21) das Ventil (15, 16) derart ansteuert, dass das druckmittelbetätigte Steuerungsorgan die Schlauchleitung vollständig verschließt, bevor eine Gasblase, die das Detektorsignal hervorgerufen hat, mit dem Blutstrom das druckmittelbetätigte Steuerungsorgan passiert.

6. Herz-Lungen-Maschine nach Anspruch 5, **dadurch gekennzeichnet, dass** der Prozessor (14) ferner die Pumpe (5) anhält.

7. Herz-Lungen-Maschine nach einem der Ansprüche 1 bis 6, **dadurch gekennzeichnet, dass** das druckmittelbetätigte Steuerungsorgan einen Kolben (30) umfasst, der von einer Feder (32) in eine Ruhelage bewegt wird und der in einem Zylinder (31) angeordnet ist, so dass der Kolben (30) entgegen der Federkraft bewegt wird, wenn dem Zylinder (31) das Druckmittel zugeführt wird, und auf die Schlauchleitung einwirkt.

8. Herz-Lungen-Maschine nach einem der Ansprüche 1 bis 7, **dadurch gekennzeichnet, dass** das Druckmittel ein Gas, insbesondere Luft, Helium oder Stickstoff ist.

9. Herz-Lungen-Maschine nach einem der Ansprüche 1 bis 8, **dadurch gekennzeichnet, dass** die Druckmittelabgabeeinrichtung (17) ein Kompressor ist.

10. Herz-Lungen-Maschine nach einem der Ansprüche 1 bis 8, **dadurch gekennzeichnet, dass** die Druckmittelabgabeeinrichtung (17) eine Druckflasche ist.

11. Herz-Lungen-Maschine nach einem der Ansprüche 1 bis 8, **dadurch gekennzeichnet, dass** die Druckmittelabgabeeinrichtung (17) ein in ein Gebäude eingebautes Druckgasversorgungsnetz ist.

## Claims

1. Heart-lung machine having at least one tube assembly (6, 10; 34), which is integrated into an extracorporeal blood circulatory system (2, 3, 4, 5, 6, 7, 8, 9, 10) of the heart-lung machine, having at least one pump (5), which conveys the blood of a patient at least sometimes through the extracorporeal circulatory system, at least one pressure agent-actuated control element (20, 21; 30, 31, 32, 33), which acts externally on the tube assembly (6, 10; 34), and a control device (11) for control of the supply of pressure agent to the control element, **characterised in that** a pressure agent having an operating pressure in the region of 2 to 10 bar is provided at the site of the pressure agent-actuatable control element.

2. Heart-lung machine according to claim 1, **characterised in that** the pressure agent supplied to the pressure agent-actuated control element has an operating pressure in the region of about 5 bar.

3. Heart-lung machine according to one of claims 1 or 2, **characterised in that** the control (11) is connected to a pressure agent release device (17), and has at least one valve device (15, 16), to which the pressure agent is supplied by the pressure agent release device, and a processor (14), which is connected to valve device (15, 16) for control thereof.

4. Heart-lung machine according to one of claims 1, 2 or 3, **characterised in that** at least one bubble detector (12, 13) is provided to detect gas bubbles in the blood stream guided in the tube assembly relative to the blood stream above the pressure agent-actuated control element (20, 21), and **in that** the processor (14) of the control (11) is connected to the bubble detector (12, 13) for receiving a detector signal in order to switch over the valve (20, 21).

5. Heart-lung machine according to claim 4, **characterised in that** when receiving a detector signal from the bubble detector (20, 21), the processor (14) controls the valve (15, 16) such that the pressure agent-actuated control element closes the tube assembly completely before a gas bubble, which has caused the detector signal, passes the pressure agent-actuated control element with the blood stream.

6. Heart-lung machine according to claim 5, **characterised in that** the processor (14) also stops the pump (5).

7. Heart-lung machine according to one of claims 1 to 6, **characterised in that** the pressure agent-actuated control element comprises a piston (30), which is moved into a rest position by a spring (32) and which is arranged in a cylinder (31), so that the piston (30) is moved against the spring force when the pressure agent is supplied to the cylinder (31), and acts on the tube assembly.

8. Heart-lung machine according to one of claims 1 to 7, **characterised in that** the pressure agent is a gas, in particular air, helium or nitrogen.

9. Heart-lung machine according to one of claims 1 to 8, **characterised in that** the pressure agent release device (17) is a compressor.

10. Heart-lung machine according to one of claims 1 to 8, **characterised in that** the pressure agent release device (17) is a compressed cylinder.

11. Heart-lung machine according to one of claims 1 to 8, **characterised in that** the pressure agent release device (17) is a compressed gas supply network installed in a building.

## Revendications

1. Un système cardiopulmonaire comprenant au moins une conduite en tuyau (6, 10 ; 34), intégrée dans un circuit sanguin (2, 3, 4, 5, 6, 7, 8, 9, 10) extracorporel du système cardio-pulmonaire, avec au moins une pompe (5) véhiculant le sang d'un patient, au moins par tronçons, à travers le circuit extracoporel, au moins un organe de commande (20, 21 ; 30, 31, 32, 33), actionné par un moyen de pression, agissant depuis l'extérieur sur la conduite en tuyau souple (6, 10 ; 34), et un dispositif de commande (11), pour la commande de l'amenée d'un moyen de pression à l'organe de commande,
**caractérisé en ce que**, sur le site de l'organe de commande pouvant être actionné par un moyen de pression, est fourni un moyen de pression, sous une pression de service dans la fourchette de 2 à 10 bar.

2. Système cardiopulmonaire selon la revendication 1, **caractérisé en ce que** le moyen de pression amené à l'organe de commande actionné par un moyen de pression présente une pression de service de l'ordre d'environ 5 bar.

3. Système cardiopulmonaire selon l'une des revendications 1 ou 2, **caractérisé en ce que** la commande (11) est reliée à un dispositif de délivrance de moyen de pression (17), et présente au moins un dispositif à soupape (15, 16), auquel le moyen de pression est amené par le dispositif de délivrance de fluide sous pression, et un processeur (14), relié au dispositif à soupape (15, 16), pour sa commande.

4. Système cardiopulmonaire selon l'une des revendications 1 ou 2 ou 3, **caractérisé en ce qu'**au moins un détecteur de bulles (12, 13) est prévu, pour constater la présence de bulles de gaz dans le flux sanguin guidé dans la conduite en tuyau souple, par rapport au flux sanguin en amont de l'organe de commande (20, 21) actionné par un moyen de pression, et **en ce que** le processeur (14) de la commande (11) est relié au détecteur de bulles (12, 13), pour recevoir un signal de détecteur afin de commuter la soupape (20, 21).

5. Système cardiopulmonaire selon la revendication 4, **caractérisé en ce que** le processeur (14), à réception d'un signal de détecteur, venant du détecteur de bulles (21), commande la soupape (15, 16), de manière que l'organe de commande actionné par un moyen de pression ferme complètement la conduite en tuyau souple, avant qu'une bulle de gaz, qui a provoqué le signal de détecteur, passe, avec le flux sanguin, par l'organe de commande actionné par un moyen de pression.

6. Système cardiopulmonaire selon la revendication 5, **caractérisé en ce que** le processeur (14) en outre stoppe la pompe (5).

7. Système cardiopulmonaire selon l'une des revendications 1 à 6, **caractérisé en ce que** l'organe de commande actionné par un moyen de pression comprend un piston (30) déplacé, par un ressort (32), en une position de repos et disposé dans un cylindre (31) de sorte que le piston (30) soit déplacé à l'encontre de la force élastique, lorsque le moyen de pression est amené au cylindre (31) et agit sur la conduite en tuyau souple.

8. Système cardiopulmonaire selon l'une des revendications 1 à 7, **caractérisé en ce que** le moyen de pression est un gaz, en particulier de l'air, de l'hélium ou de l'azote.

9. Système cardiopulmonaire selon l'une des revendications 1 à 8, **caractérisé en ce que** le dispositif de délivrance de moyen de pression (17) est un commpresseur.

10. Système cardiopulmonaire selon l'une des revendications 1 à 8, **caractérisé en ce que** le dispositif de délivrance de moyen de pression (17) est une bouteille à pression.

11. Système cardiopulmonaire selon l'une des revendications 1 à 8, **caractérisé en ce que** le dispositif de délivrance de moyen de pression (17) est un réseau d'alimentation en gaz sous pression, intégré dans un bâtiment.
